# FASCICULE DE BREVET EUROPEEN

(11) **EP 1 507 792 B1**
(45) Date de publication et mention de la délivrance du brevet: **06.09.2006**
(21) Numéro de dépôt: 03755166.0
(22) Date de dépôt: 15.05.2003
(51) Int. Cl.: C07K 1/107, C07K 17/02, G01N 33/543

(54) **DISPOSITIF DE PRESENTATION DE PEPTIDES OU DE PROTEINES, SON PROCEDE DE PREPARATION ET SES UTILISATIONS.**
VORRICHTUNG VON PEPTID- ODER PROTEINDARSTELLUNG, VERFAHREN ZU DEREN HERSTELLUNG UND DEREN VERWENDUNG
DEVICE FOR THE PRESENTATION OF PEPTIDES OR PROTEINS, METHOD FOR THE PREPARATION AND USE THEREOF

(30) Priorité: 28.05.2002 FR 0206489
(43) Date de publication de la demande: 23.02.2005
(73) Titulaire: Sedac Therapeutics, 59120 Loos (FR); Centre National de la Recherche Scientifique, 75016 Paris (FR); UNIVERSITE DE LILLE 2, 59800 Lille (FR)
(72) Inventeur: MELNYK, Oleg, F-59112 Annoeullin (FR); DUBURCQ, Xavier, F-59800 Lille (FR); GRAS-MASSE, Hélène, F-59710 Merignies (FR); OLIVIER, Christophe, F-59000 Lille (FR); ZHOU, Fengling, F-59000 Lille (FR); AURIAULT, Claude, F-59310 Nomain (FR); BOUZIDI, Ahmed, F-59112 Annoeullin (FR)
(74) Mandataire: Goulard, Sophie
(86) Numéro de dépôt international: PCT/FR2003/001471
(87) Numéro de publication internationale: WO 2003/099855

(56) Documents cités:
- WO-A-00/63701
- WO-A-01/42495
- WO-A-02/097442
- O MELNYK ET AL.: "Peptide arrays for highly sensitive and specific antibody-binding fluorescence assays" BIOCONJUGATE CHEMISTRY., vol. 13, no. 4, avril 2002 (2002-04), pages 713-720, XP002237583 AMERICAN CHEMICAL SOCIETY, WASHINGTON., US ISSN: 1043-1802

## Description

La présente Invention est relative à un dispositif de présentation de polypeptides, à son procédé de préparation ainsi qu'à ses utilisations, notamment comme outil de diagnostic (puce à polypeptides) pour la détection miniaturisée de molécules structurellement ou fonctionnellement complémentaires desdits polypeptides, notamment d'anticorps.

Au sens de la présente Invention, le terme "puce à polypeptides" correspond aux termes anglais "peptide arrays", "peptide microarrays", "peptide chips", "protein chips" ou encore "protein arrays", couramment utilisés dans la littérature.

On connaît actuellement un grand engouement pour l'utilisation de puces à polypeptides permettant la détection, dans divers milieux biologiques liquides, d'anticorps ou de parties spécifiques de ceux-ci, d'antigènes (notamment viraux, bactériens ou parasitaires), de récepteurs, de séquences responsables de la liaison à une molécule (enzyme, récepteur, anticorps), l'étude de la spécificité d'enzymes ou encore la mise au point de récepteurs artificiels.

Or, dans ce cadre particulier, il est primordial de pouvoir disposer de puces à polypeptides présentant un certain nombre de qualités.

Ces puces doivent en particulier permettre l'immobilisation reproductible des sondes, dans la mesure où une immobilisation reproductible est conditionnelle d'une détection elle-même reproductible. Au sens de la présente Invention, on entend par sonde, tout polypeptide qui est déposé sur la surface d'un support et qui sert à la capture de cibles présentes dans un milieu biologique, ces sondes étant spécifiques des cibles à détecter.

Ces puces doivent également permettre la détection sensible des cibles ou des récepteurs complémentaires contenus dans le milieu biologique. La sensibilité de la détection dépend du taux d'immobilisation, du taux de capture et de la méthode de détection d'un signal, mais aussi et surtout du niveau du bruit de fond (signal non spécifique). Une diminution du bruit de fond améliore le rapport signal/bruit. En effet, dans un dispositif dans lequel on détecte la présence d'espèces biologiques au voisinage de la surface, le bruit de fond vient essentiellement de l'adsorption non spécifique de molécules autres que les espèces biologiques d'intérêt que l'on souhaite détecter et qu'il convient par conséquent de limiter. L'idéal est donc d'obtenir un dispositif qui possède un bruit de fond très bas et une intensité de détection du signal élevé.

Par ailleurs, d'un point de vue industriel, il est intéressant de pouvoir disposer de dispositifs pouvant être préparés de façon simple et présentant une excellente stabilité au stockage avant utilisation.

A l'heure actuelle, il existe principalement deux grandes méthodes de préparation de puces à polypeptides utilisant des polypeptides préparés *ex situ.*

La première grande méthode consiste à lier les polypeptides de manière covalente à une surface solide comme une surface de verre ou de polymère organique. C'est ainsi qu'il a déjà été proposé par exemple dans l'article de G. MacBeath *et al.,* Science, 2000, **289,** 1760-1763, un procédé de préparation consistant à immobiliser les polypeptides par l'intermédiaire d'une liaison imine résultant de la réaction entre une fonction amine des polypeptides et une fonction aldéhyde d'un support silanisé. Ce procédé nécessite ensuite une étape de réduction in situ de la fonction imine, par exemple par du borohydrure de sodium afin de stabiliser le lien polypeptide-surface. L'inconvénient majeur de cette approche est la nécessité de réaliser des étapes chimiques pour lier le polypeptide à la surface du support, qui peuvent notamment entraîner, dans certains cas, une dégradation du polypeptide mis en oeuvre. De plus de telles réactions compliquent de façon non négligeable le procédé de fabrication des puces à polypeptides.

La deuxième grande méthode consiste à immobiliser les polypeptides par adsorption sur une surface sans établir de lien covalent. Cette méthode présente bien évidemment l'avantage d'être plus simple d'un point de vue industriel puisqu'elle ne met pas forcément en jeu d'étape chimique de fonctionnalisation des polypeptides. C'est ainsi que l'article de Falipou S. *et al.,* Bioconjugate Chem., 1999, **10**, 346-353, décrit une méthode selon laquelle des billes de SiO₂ ou des lames de verre sont silanisées avec du 3-cyanopropyldiméthylchlorosilane de façon à permettre l'immobilisation d'anticorps (protéines glycosylées), la fixation non covalente se faisant, dans ce cas, par l'intermédiaire des fonctions hydroxyle des parties glycosylées. Cette méthode a cependant l'inconvénient d'être limitée aux protéines glycosylées. Il a par ailleurs déjà été proposé, notamment dans la demande internationale WO 00/63701, d'immobiliser des polypeptides sur des surfaces de verre recouvertes par un polymère cationique tel que la polylysine, au moyen de liaisons électrostatiques. Cependant, avant même l'immobilisation des polypeptides, les propriétés des lames de verre ainsi préparées évoluent dans le temps. Aussi, les dispositifs fabriqués à partir de ce genre de support sont instables dans le temps (variation du signal en fonction du degré de vieillissement du dispositif). Enfin, ces surfaces conduisent à un niveau de bruit de fond important (Haab BB. *et al.,* Genome Biology, 2001, research 0004.1-13).

Les Inventeurs se sont donc donnés pour but de remédier à l'ensemble des problèmes rencontrés avec les dispositifs de l'art antérieur décrits ci-dessus et de pourvoir à des dispositifs de présentation de polypeptides qui soient stables dans le temps (au moins 3 mois de vieillissement en conditions accélérées, ce qui correspond à 12 mois de vieillissement à température ambiante), simples à fabriquer et à mettre en oeuvre, applicables à toute sorte de polypeptides sans qu'il soit nécessaire de passer par des étapes de fonctionnalisation de ceux-ci et permettant la détection sensible et reproductible d'un signal avec un très faible bruit de fond.

La présente Invention a donc pour premier objet un dispositif de présentation de polypeptides caractérisé par le fait qu'il comprend au moins un support solide fonctionnalisé par des groupements semicarbazides sur lesquels sont adsorbés lesdits polypeptides.

Alors que les surfaces comportant des groupements semicarbazides sont habituellement utilisées pour immobiliser des biomolécules comme des acides nucléiques fonctionnalisés par des groupements benzaldéhydes (Podyminogin MA *et al.,* Nucleic Acid Research, 2001, **29,** 5090-5098) ou des polypeptides modifiés par des fonctions α-oxo aldéhydes (Demande internationale WO 01/42495 ou Melnyk, O. *et al.,* Bioconjugate Chem., 2002, **13,** 713-720) par formation d'une liaison covalente de type semicarbazone, les Inventeurs ont constaté, de manière surprenante, que l'utilisation de ces surfaces permet également d'immobiliser des polypeptides par simple adsorption, de façon durable et stable dans le temps, sans qu'il soit nécessaire de les fonctionnaliser préalablement.

Ainsi, la simple adsorption de polypeptides sur un tel support permet d'accéder à des dispositifs qui sont stables dans le temps (au moins 12 mois à température ambiante), simples à fabriquer et à mettre en oeuvre, selon une méthodologie applicable à toute sorte de polypeptides (sans ajout de réactifs chimiques, hormis les tampons classiquement utilisés pour solubiliser des polypeptides) et qui permettent la détection sensible et reproductible d'un signal avec un très faible bruit de fond.

Au sens de la présente Invention, le terme général de "polypeptides" désigne l'ensemble des peptides comprenant au moins deux acides aminés (de la série L ou D, alpha-aminoacides, beta-aminoacides, alpha-hydrazinoacides, alpha-aminoacides protéinogéniques ou non), les peptidomimétiques (mimes de structures secondaires, mimes de coude beta par exemple), les protéines et les fragments de protéines.

Les polypeptides adsorbés à la surface du dispositif conforme à la présente Invention peuvent être des polypeptides d'extraction ou recombinants, sans contrainte quant à leur structure (polypeptides avec ou sans modification post-traductionnelle). Il peut également s'agir de polypeptides synthétiques portant des modifications diverses, comme par exemple un groupement polyéthylèneglycol ou des groupements chimiques inertes vis-à-vis des groupements semicarbazides de surface, tels que par exemples des groupements semicarbazone et hydrazone.

Le dépôt des polypeptides sur le support semicarbazide s'accompagne de leur adsorption spontanée au support.

Tous les supports solides pouvant être fonctionnalisés par un groupement semicarbazide sont utilisables selon l'Invention. Parmi de tels supports, on peut en particulier citer les matériaux organiques et inorganiques, par exemple choisis parmi le verre, le silicium et ses dérivés et les polymères synthétiques.

Les supports fonctionnalisés par des groupements semicarbazides peuvent être imprimés par exemple avec un "spotteur" à aiguilles.

Ainsi, la stratégie d'immobilisation :
- est simple au niveau expérimental et d'une grande reproductibilité ;
- s'applique à tout type de polypeptide non fonctionnalisé ou fonctionnalisé par un groupement chimiquement inerte vis-à-vis des groupements semicarbazides du support ;
- met en jeu des surfaces fonctionnalisées par une fonction stable, non hydrolysable ;
- permet d'obtenir une grande densité d'adsorption des polypeptides à la surface du support ce qui assure un rapport signal/bruit de fond très élevé (typiquement le bruit de fond représente 0,1 % du signal détecté).

La qualité de l'adsorption des polypeptides sur le support (densité, homogénéité) peut être contrôlée par sa capacité à fixer une sonde peptidique de synthèse fluorescente.

L'Invention a également pour objet un procédé de préparation des dispositifs de présentation de polypeptides tels que décrit ci-dessus, comprenant les étapes suivantes :
- la fonctionnalisation d'un support solide par des groupements semicarbazides, et
- le dépôt sous forme de spots d'échantillons de polypeptides et leur adsorption sur le support ainsi fonctionnalisé.

Selon une première variante de ce procédé, la fonctionnalisation du support comprend :
- l'introduction d'une fonction amine par une réaction de silanisation du support,
- la transformation de la fonction amine en fonction isocyanate,
- la réaction de la fonction isocyanate avec un dérivé d'hydrazine pour former le groupement semicarbazide.

Selon une seconde variante de ce procédé, la fonctionnalisation du support est effectuée en une seule étape par réaction du support avec un silane portant un groupement semicarbazide.

L'étape de dépôt des polypeptides comprend de préférence :
- la préparation de solutions de polypeptides dans un tampon, à une concentration comprise entre 10 mg/ml et 0,01 mg/ml ;
- leur distribution dans un récipient approprié à leur prélèvement, de type puits de plaque de microtitration ;
- leur prélèvement à l'aide d'un appareil de prélèvement manuel ou automatisé, par exemple de type "spotteur" ;
- leur dépôt sur le support semicarbazide ; et éventuellement
- la saturation du support.

A la fin de leur préparation, les dispositifs conformes à l'Invention, peuvent être directement utilisés ou bien stockés à température ambiante, à l'abri de la lumière et de la poussière.

L'Invention a également pour objet l'utilisation des dispositifs de présentation de polypeptides comme "puces à polypeptides", en tant qu'outil de diagnostic miniaturisé et hautement parallèle, ou pour la détection d'un risque lors d'une transfusion ou d'un don d'organe.

Les dispositifs conformes à la présente Invention peuvent également être utilisés comme "puces à polypeptides" pour le sérotypage ou le criblage d'épitopes.

Ces utilisations mettent en jeu la détection de réponses de type antigène-anticorps par l'utilisation de réactifs marqués, fluorescents, radioactifs ou marqués chimiquement.

Les dispositifs conformes à la présente Invention peuvent également être utilisés comme puces à polypeptides pour la quantification de protéines dans des milieux biologiques complexes.

Enfin, les dispositifs conformes à la présente Invention peuvent aussi être utilisés pour l'analyse des relations entre molécules biologiques peptidiques, de type ligand-récepteur.

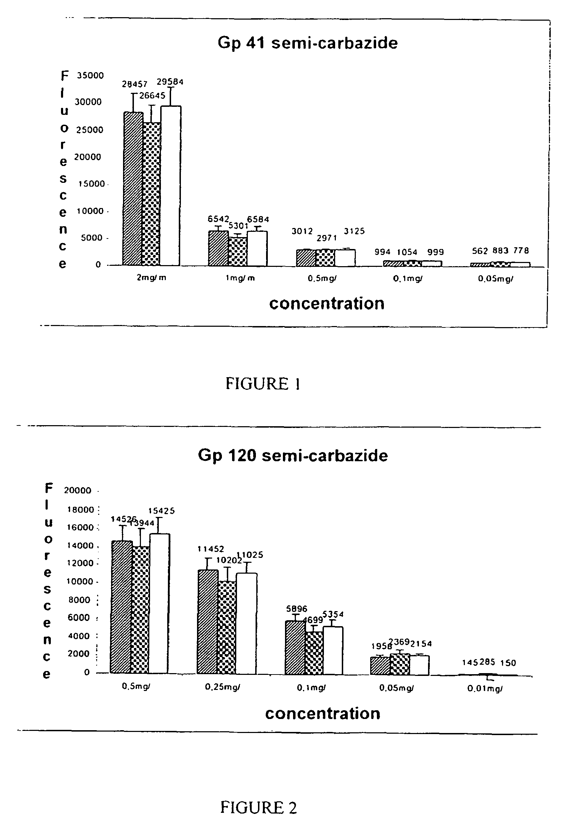

Outre les dispositions qui précèdent, l'Invention comprend encore d'autres dispositions qui ressortiront de la description qui va suivre, qui se réfère à un exemple de préparation de lames de verres fonctionnalisées par des groupements semicarbazides, à un exemple détaillant le protocole d'adsorption de polypeptides sur des lames de verre semicarbazides et l'utilisation des dispositifs correspondants ainsi obtenus, d'utilisation des dispositifs de l'Invention pour le sérodiagnostic de l'hépatite B, de l'hépatite C et du SIDA, d'études de la stabilité de ces dispositifs, d'étude de multi séro-détection et de détection de l'antigène de surface de l'hépatite B, ainsi qu'aux figures 1 à 5 annexées dans lesquelles :
- les figures 1 et 2 représentent le signal de fluorescence mesuré en fonction de la concentration en antigènes, après 0, 1 ou trois mois de vieillissement de lames semicarbazides sur lesquelles sont adsorbés des antigènes du virus du SIDA ;
- les figures 3 et 4 représentent le signal de fluorescence mesuré pour des lames aminées sur lesquelles ont été fixés des antigènes du virus du SIDA en fonction de la concentration, et ce après 0 et 1 mois de vieillissement accéléré ;
- la figure 5 représente la fluorescence mesurée en fonction de la quantité d'un antigène HBs recombinant dans un test de séro-détection de l'antigène de surface de l'hépatite B.

### EXEMPLE 1 : FONCTIONNALISATION DE LAMES DE VERRES PAR DES GROUPEMENTS SEMICARBAZIDES

Cette fonctionnalisation a été réalisée selon deux variantes.

### 1) Première variante

### a) Etape A : lavage, décapage et silanisation

Des lames porte-objets commerciales (Esco) pré-nettoyées, à bords rodés et à marge dépolie sont plongées dans une solution constituée d'un mélange d'eau oxygénée et d'acide sulfurique (50/50, v/v) pendant une nuit. Des rinçages préalables de trois minutes sont effectués par de l'eau désionisée (3 fois) puis par du méthanol (1 fois), avant de plonger les lames dans un bain à 3% d'aminopropyltriméthoxysilane dans du méthanol à 95 % pendant 30 minutes sous ultrasons. Les lames sont ensuite rincées successivement par des bains de 3 minutes dans du méthanol (1 fois), par de l'eau désionisée (2 fois) et enfin par du méthanol (1 fois). Les lames sont alors égouttées pendant quelques minutes, séchées pendant 15 minutes dans une étuve à 110°C, puis stockées dans un dessiccateur sous vide.

### b) Etape B : Formation d'un isocyanate

Les lames précédemment silanisées sont plongées pendant 2 heures dans une solution de 1,2-dichloroéthane contenant du triphosgène (100 mmol/l) et de la diisopropyléthylamine (DIEA) (800 mmol/l).

### c) Etape C : Fonctionnalisation par des groupements semicarbazides

Les lames obtenues à l'étape b) ci-dessus sont ensuite rapidement égouttées avant d'être directement plongées dans une solution contenant du 9-fluorénylméthoxycarbonyl-NH-NH₂ (Fmoc-NH-NH₂), préalablement préparé selon Zhang *et al.,* Anal. Biochem., 1991, **195,** 160-170, à 22 mmol/l dans du diméthylformamide (DMF) et traitées pendant 2 heures aux ultrasons. Les lames sont ensuite rincées successivement par deux bains de 3 minutes dans du DMF.

### d) Etape D : Déprotection

Les lames précédemment obtenues à l'étape c) ci-dessus sont plongées dans une solution de DMF contenant de la pipéridine (0,2 % en volume) et du 1,8-diazabicyclo[5.4.0]-undec-7-ène (DBU) (2 % en volume) pendant 30 minutes. Les lames sont alors rincées successivement par des bains de 3 minutes dans du DMF (1 fois), par de l'eau désionisée (2 fois) et enfin par du méthanol (1 fois) avant d'être séchées et stockées dans un dessiccateur sous vide.

### 2) Deuxième variante

Les étapes de silanisation et de fonctionnalisation par les groupements semicarbazides sont couplées en une seule réaction, par préparation préalable du réactif.

### a) Etape A : préparation du réactif de silanisation contenant le groupement semicarbazide protégé (Fmoc-NH-NH-CO-NH-(CH₂)₃-Si-(OEt₃)

515 mg de Fmoc-NH-NH₂ (2,03 mmoles) sont mis en suspension dans 15 ml d'éthanol absolu. Le mélange est porté au reflux (75-80°C). 570 ml d'isocyanopropyltriéthoxysilane (2,28 mmoles, 1,2 eq) sont alors ajoutés en une fois. Après disparition de la suspension (15-20 minutes), l'éthanol est évaporé. Le solide blanc obtenu est dissous dans un minimum de dichlorométhane sec, puis précipité grâce à du pentane sec. Après filtration sous argon, on récupère 841 mg (83 %) d'un solide pur.

### b) Etape B : Préparation des lames

Des lames porte-objets commerciales (Esco) pré-nettoyées, à bords rodés et à marge dépolie sont plongées dans une solution constituée d'un mélange d'eau oxygénée et d'acide sulfurique (50/50, v/v) pendant une nuit. Les lames sont ensuite rincées sous agitation dans les bains successifs suivants : eau désionisée (3 fois 3 minutes), éthanol absolu (1 fois 3 minutes) puis séchées à la pompe à palettes.

Les lames sont ensuite plongées pendant 2 heures dans une solution à 1 mg/ml du réactif de silanisation préparé ci-dessus à l'étape a) dans un mélange de tetrahydrofurane (THF) à 10 % dans du toluène à 47°C et sous ultrasons. Les lames sont ensuite rincées sous agitation dans du toluène (2 fois 3 minutes) avant d'être égouttées, puis séchées pendant 15 minutes dans une étuve à 120°C et stockées dans un dessiccateur sous vide.

### c) Etape C : Déprotection

Les lames précédemment obtenues ci-dessus à l'étape b) sont plongées dans une solution de DMF pendant 3 minutes avant d'être mises sous agitation dans un bain contenant de la pipéridine (0,2 % en volume) et du diazabicyclo-undecène (2 % en volume) dans du DMF pendant 30 minutes sous agitation. Les lames sont alors rincées successivement par des bains de 3 minutes dans du DMF (1 fois), dans de l'eau désionisée (2 fois) et enfin dans du méthanol (1 fois) avant d'être séchées et stockées dans un dessiccateur sous vide.

Les lames ainsi préparées sont prêtes à être utilisées pour adsorber des polypeptides.

### EXEMPLE 2 : PROTOCOLE D'UTILISATION DES LAMES SEMICARBAZIDES

### 1) Adsorption des polypeptides sur les lames

Des solutions de peptides synthétiques ou de protéines recombinantes (antigènes) sont diluées, entre 0,1 et 10 mg/ml suivant la protéine utilisée, dans un tampon phosphate (pH 7,4) ou dans un tampon carbonate/bicarbonate (pH 9,2) puis sont réparties dans les puits d'une plaque ELISA de 96 puits. Un volume minimum de 20 µl d'échantillon par puits est nécessaire pour réaliser l'étape d'impression des lames.

La plaque est alors installée sur un spotteur automatique à 4 aiguilles (par exemple de type MWG 417 Arrayer de la société Affymetrix). On appelle spotteur l'appareil permettant de faire les dépôts de solutions d'antigènes.

Les lames sont alors imprimées avec les solutions de polypeptides selon un schéma pré-établi. Les aiguilles sont ensuite lavées abondamment selon les recommandations du fabricant.

### 2) Stockage des lames

Les lames ainsi imprimées sont conservées à température ambiante, dans un placard fermé, à l'abri de la lumière et de la poussière. La stabilité des lames a été étudiée (voir exemple 6 ci-après). Les résultats montrent que les propriétés des lames ainsi imprimées ne sont pas altérées durant un stockage de trois mois à 37°C en atmosphère humide (conditions de vieillissement accéléré).

### 3) Révélation des lames

Les lames imprimées sont tout d'abord soumises à une étape de sonication pendant 1 heure dans une solution de saturation : tampon phosphate additionné de 1,8 % de NaCl, pH 7,4 (solution de PBS) + 0,1 % de Tween® 20 + 5 % de lait écrémé).

Les lames sont ensuite soumises à trois lavages successifs avec une solution de PBS en présence de 0,05 % de Tween® 20.

Les lames sont alors incubées pendant 45 minutes à 37°C en présence de 100 µl de sérum de patients, dilués au 1/50^{ème} dans un tampon de dilution (solution de PBS + 0,1 % de Tween® 20 + 5 % de lait écrémé) sous une lamelle de verre couvre-objet (24x60 mm). L'incubation se fait en atmosphère humide.

Suite à cette incubation, trois lavages successifs des lames avec une solution de PBS additionné de 0,05 % de Tween® 20 sont alors effectués.

L'étape de détection des anticorps de patients sur les polypeptides adsorbés sur les lames est effectuée par fixation, sur ceux-ci d'anticorps anti-Ig humaines fluorescents : 100 µl de solution d'anticorps anti-IgG-A-M humaines marqués à la rhodamine (TRITC) (Jackson ImmunoResearch Laboratories, Baltimore, USA), diluée au 1/100^{ème} dans du tampon de dilution (solution de PBS + 0,1 % de Tween® 20 + 5 % de lait écrémé). On recouvre alors chaque lame avec une lamelle de verre (24x60 mm) et on laisse incuber pendant 45 minutes à 37°C sous atmosphère humide.

Suite à cette incubation, une série de 3 lavages successifs des lames avec une solution de PBS additionné de 0,05 % de Tween® 20 est alors effectuée. Ces lavages sont suivis par un rinçage des lames à l'aide d'eau distillée et par un séchage des lames à l'éthanol.

La lecture des lames est alors effectuée par mesure de la fluorescence émise à l'aide d'un scanner à lame (Affymetrix 418 Array Scanner), à deux puissances différentes (P35/PMT 50 ou P55/PMT 70). La quantification de la fluorescence émise est alors effectuée à l'aide du logiciel Scanalyse® (Stanford-University Software). Sauf indication contraire, les résultats présentés dans les Tableaux ci-après ont été obtenus à la puissance P35/PMT 50.

Tous les exemples qui suivent ont été réalisés en suivant ce protocole.

### EXEMPLE 3 : UTILISATION DE PUCES A POLYPEPTIDES SELON L'INVENTION POUR LE SÉRODIAGNOSTIC DE L'HÉPATITE B

### 1) Matériel et méthodes

Tous les dépôts ont été effectués en double sur des lames telles que préparées ci-dessus à l'exemple 1, à l'aide d'un spotteur MWG 417 Arrayer (Affymetrix). L'espacement entre les dépôts est de 375 µm, le contrôle de l'efficacité du lavage des aiguilles se fait par dépôt d'eau, ce qui ne révèle en fluorescence aucune trace d'antigène.

Dans cet exemple, les lames de verre ont été imprimées avec différents antigènes du virus de l'hépatite B. Les termes surface (HBs), core (HBc) ou (Hbe) correspondent à différentes régions de ce virus. 20 dépôts par antigène et par concentration ont été réalisés.
- Antigène HBs, deux lots ont été utilisés :
   - HBs Lot a : antigène commercialisé par la société Advanced Immuno Chemical sous la référence A1-HS7, à 3 mg/ml.
   - HBs Lot b *: 5 mg/ml.
- Antigène Hbe *: 10 mg/ml.
- Antigène HBc *: 10 mg/ml.
- Témoin positif : protéine A
   * Les antigènes HBV (HBs lot b, Hbe, HBc) et HCV ont été produits par "The Hepatopathy Research Institute", Hôpital pédiatrique de Beijing, (Beijing, Chine).

Des sérums de patients dont la sérologie est connue (c'est-à-dire vérifiée avec un test ELISA de référence) ont été analysés en utilisant les puces conformes à l'invention.

La répartition des sérums était la suivante :
- Sérodiagnostic Ag HBs avec le lot a : 40 sérums référencés positifs et 40 sérums référencés négatifs ;
- Sérodiagnostic Ag HBs avec le lot b : 60 sérums référencés positifs et 40 sérums référencés négatifs ;
- Sérodiagnostic Hbe : 16 sérums référencés positifs et 16 sérums référencés négatifs ;
- Sérodiagnostic Hbc : 60 sérums référencés positifs et 40 sérums référencés négatifs.

### 2) Résultats

Les résultats obtenus pour les sérums positifs sont reportés dans le Tableau 1 ci-après. Dans ce tableau, les valeurs données correspondent à la valeur de la fluorescence moins le bruit de fond moyen de la lame.

**TABLEAU I**

| Sérum | Test ELISA de référence | | | Test sur biopuces (quantité de fluorescence) | | | |
|---|---|---|---|---|---|---|---|
| | HBs | HBe | HBc | HBs Lot a | HBs Lot b | HBe | HBc |
| 1 | + | + | + | 5482 | 5548 | 10254 | 15049 |
| 2 | + | nt * | + | 4521 | 7845 | nt | 16804 |
| 3 | + | nt | + | 2899 | 5784 | nt | 25014 |
| 4 | + | + | + | 4503 | 9854 | 5784 | 11568 |
| 5 | + | + | + | 4310 | 8547 | 14215 | 19580 |
| 6 | + | nt | + | 6854 | 7548 | nt | 28954 |
| 7 | + | + | + | 5871 | 10055 | 8457 | 9109 |
| 8 | + | nt | + | 3771 | 7156 | nt | 14201 |
| 9 | + | + | + | 4587 | 8457 | 11451 | 21131 |
| 10 | + | nt | + | 5684 | 11784 | nt | 9605 |
| 11 | + | + | + | 4089 | 9524 | 10254 | 15460 |
| 12 | + | nt | + | 3854 | 9995 | nt | 12541 |
| 13 | + | + | + | 2998 | 8457 | 11254 | 47988 |
| 14 | + | nt | + | 7033 | 9854 | nt | 25153 |
| 15 | + | + | + | 5714 | 12541 | 11116 | 27405 |
| 16 | + | nt | + | 3601 | 6985 | nt | 18954 |
| 17 | + | nt | + | 4707 | 7722 | nt | 14521 |
| 18 | + | + | + | 5515 | 8146 | 7895 | 25254 |
| 19 | + | nt | + | 6939 | 9524 | nt | 10939 |
| 20 | + | nt | + | 4196 | 10091 | nt | 34137 |
| 21 | + | nt | + | 5553 | 9104 | nt | 36387 |
| 22 | + | nt | + | 4357 | 9182 | nt | 22703 |
| 23 | + | + | + | 4007 | 10541 | 9548 | 31225 |
| 24 | + | nt | + | 4739 | 16608 | nt | 36695 |
| 25 | + | nt | + | 3958 | 12541 | nt | 23552 |
| 26 | + | nt | + | 5270 | 8620 | nt | 11251 |
| 27 | + | nt | + | 3254 | 9288 | nt | 57835 |
| 28 | + | + | + | 4194 | 11415 | 7854 | 18828 |
| 29 | + | nt | + | 3997 | 7698 | nt | 28461 |
| 30 | + | nt | + | 5980 | 8145 | nt | 35261 |
| 31 | + | nt | + | 3215 | 9354 | nt | 15854 |
| 32 | + | nt | + | 4254 | 7125 | nt | 28554 |
| 33 | + | + | + | 5412 | 16859 | 5896 | 26551 |
| 34 | + | nt | + | 3984 | 8327 | nt | 29376 |
| 35 | + | nt | + | 4521 | 10270 | nt | 15189 |
| 36 | + | nt | + | 4879 | 7458 | nt | 29276 |
| 37 | + | nt | + | 5895 | 17791 | nt | 25654 |
| 38 | + | nt | + | 4987 | 13232 | nt | 29854 |
| 39 | + | nt | + | 6587 | 14587 | nt | 21900 |
| 40 | + | nt | + | 3254 | 9587 | nt | 15854 |
| 41 | + | nt | + | nt | 9854 | nt | 25044 |
| 42 | + | nt | + | nt | 9968 | nt | 19578 |
| 43 | + | + | + | nt | 13263 | 4985 | 25654 |
| 44 | + | nt | + | nt | 12335 | nt | 21066 |
| 45 | + | + | + | nt | 11009 | 14882 | 25654 |
| 46 | + | nt | + | nt | 14528 | nt | 19850 |
| 47 | + | nt | + | nt | 7458 | nt | 9854 |
| 48 | + | nt | + | nt | 8954 | nt | 14592 |
| 49 | + | nt | + | nt | 9958 | nt | 15253 |
| 50 | + | + | + | nt | 10499 | 7180 | 12454 |
| 51 | + | nt | + | nt | 8521 | nt | 14254 |
| 52 | + | nt | + | nt | 8637 | nt | 21548 |
| 53 | + | nt | + | nt | 7410 | nt | 35689 |
| 54 | + | nt | + | nt | 9587 | nt | 14587 |
| 55 | + | nt | + | nt | 5985 | nt | 16548 |
| 56 | + | + | + | nt | 8457 | 4958 | 25415 |
| 57 | + | nt | + | nt | 9485 | nt | 39548 |
| 58 | + | nt | + | nt | 8567 | nt | 9854 |
| 59 | + | nt | + | nt | 15421 | nt | 12458 |
| 60 | + | nt | + | nt | 8214 | nt | 19874 |
| Valeur seuil | | | | 421 | 784 | 712 | 854 |

| | | | | | | | |
|---|---|---|---|---|---|---|---|
| * : nt signifie non testé | | | | | | | |

La valeur seuil (VS) est calculée comme la moyenne du signal de 20 sérums négatifs moins le bruit de fond, auquel est additionné 3 fois l'écart type calculé sur ces valeurs de sérums négatifs. Tous les sérums référencés négatifs ont donné une valeur de fluorescence inférieure à la valeur seuil et ont donc bien été trouvés négatifs en mettant en oeuvre les lames conformes à l'Invention.

L'ensemble de ces résultats montre que l'utilisation de puces à polypeptides conformes à l'Invention permet un sérodiagnostic 100 % sensible et spécifique pour tous les antigènes de l'hépatite B testés sur les différents sérums. En effet, tous les sérums répertoriés positifs ont été détectés, alors qu'aucun des sérums répertoriés négatifs n'a été trouvé faussement positif par cette méthode.

### EXEMPLE 4 : UTILISATION DE PUCES A POLYPEPTIDES SELON L'INVENTION POUR LE SÉRODIAGNOSTIC DE L'HÉPATITE C

### 1) Matériel et méthodes

Selon la méthode décrite ci-dessus à l'exemple 3, des lames de verre ont été imprimées avec différents antigènes du virus de l'hépatite C, à différentes concentrations. Les termes NS3, NS4 ou core correspondent aux différentes régions du virus de l'hépatite C. Dans cet exemple, la protéine A a été utilisée à titre de témoin positif.
- HCV Lot 1 correspondant à l'antigène entier : dépôts à 0,5 mg/ml, 0,1 mg/ml et 0,05 mg/ml ;
- HCV Lot 2 correspondant à l'antigène entier : dépôts à 2 mg/ml, 1 mg/ml et 0,5 mg/ml ;
- HCV Ag core : dépôts à 1 mg/ml, 0,5 mg/ml et 0,1 mg/ml ;
- HCV Ag NS3 Lot 3 : Dépôt à 1 mg/ml, 0,5 mg/ml et 0,1 mg/ml ;
- HCV Ag NS4 Lot 5 : Dépôts à 0,4 mg/ml et 0,1 mg/ml ;

100 sérums référencés positifs par un test classique EIA Abbot 3.0 (méthode de type ELISA) et 30 sérums référencés négatifs par la même méthode ont été testés dans cet exemple.

### 2) Résultats

Les résultats ont permis de déterminer les concentrations optimales à utiliser pour chaque antigène :
- HCV Lot 2 : 2 mg/ml
- HCV Ag NS3 Lot 3 : 0,5 mg/ml
- HCV Ag NS4 Lot 5: 0,4 mg/ml
- HCV Ag core : 1 mg/ml

Les résultats obtenus figurent dans le Tableau II ci-après. Dans ce tableau, les valeurs données correspondent à la valeur de la fluorescence moins le bruit de fond moyen de la lame.

**TABLEAU II**

| Sérum | RIBA | | | | Abbott | HCV Lot 1 | HCV Lot 2 | HCV Ag core | NS4 Ag HCV | NS3 Ag HCV |
|---|---|---|---|---|---|---|---|---|---|---|
| | NS5 | Core | NS3 | NS4 | | 0,5 mg/ml | 2 mg/ml | 1 mg/ml | 0,4 mg/ml | 0,5 mg/ml |
| 1 | | +++ | ++ | ++ | 117 | 11254 | 35268 | 17854 | 6254 | 13215 |
| 2 | - | - | - | - | 1 | 25 | 156 | 0 | 0 | 0 |
| 3 | | - | +++ | +++ | 125 | 11245 | 39421 | 0 | 10545 | 21514 |
| 4 | | - | ++ | + | 50 | 10524 | 32157 | 0 | 5214 | 8954 |
| 5 | | - | +++ | +++ | ? | 14521 | 39564 | 0 | 11245 | 15462 |
| 6 | 0,5+ | - | - | 0,5+ | 2 | 331 | 1085 | 0 | 312 | 0 |
| 7 | | ++ | + | + | 30 | 8547 | 15242 | 15421 | 5985 | 9584 |
| 8 | | - | ++ | - | 1 | 456 | 1025 | 0 | 0 | 452 |
| 9 | | - | - | - | 2 | 42 | 135 | 0 | 0 | 0 |
| 10 | | +++ | ++ | ++ | 112 | 14215 | 38546 | 21542 | 12352 | 20135 |
| 11 | | +++ | ++ | ++ | 46 | 17548 | 41526 | 23215 | 17548 | 21524 |
| 12 | + | 0,5+ | - | - | 7 | 925 | 3854 | 4162 | 0 | 0 |
| 13 | | ++ | + | ++ | 105 | 14582 | 35216 | 18546 | 13250 | 15468 |
| 14 | 0,5+ | - | 0,5+ | - | 1 | 495 | 1958 | 0 | 0 | 568 |
| 15 | | +++ | ++ | 0,5+ | ? | 10254 | 29856 | 21452 | 2154 | 7548 |
| 16 | | ++ | +++ | +++ | 130 | 15429 | 32964 | 29584 | 12496 | 16548 |
| 17 | | - | - | - | 1 | 29 | 107 | 0 | 0 | 0 |
| 18 | | - | - | - | 2 | 36 | 185 | 0 | 0 | 0 |
| 19 | | +++ | +++ | +++ | 113 | 17859 | 39520 | 28964 | 15421 | 19548 |
| 20 | | ++ | +++ | +++ | 100 | 21524 | 451214 | 31250 | 19854 | 20482 |
| 21 | | +++ | +++ | + | 74 | 19854 | 35219 | 28546 | 5485 | 17458 |
| 22 | | 0,5+ | + | 0,5+ | ? | 512 | 1021 | 456 | 651 | 325 |
| 23 | | +++ | ++ | 0,5+ | 80 | 15214 | 38546 | 21549 | 542 | 12415 |
| 24 | | - | ++ | + | 25 | 5684 | 12409 | 0 | 4632 | 6895 |
| 25 | | - | - | - | 1 | 47 | 146 | 0 | 0 | 0 |
| 26 | | - | - | - | 73 | 28 | 134 | 0 | 0 | 0 |
| 27 | | +++ | +++ | ++ | 78 | 18549 | 39507 | 25348 | 8549 | 17965 |
| 28 | | +++ | ++ | ++ | ? | 21549 | 41305 | 29846 | 14528 | 22304 |
| 29 | | - | - | - | 1 | 51 | 152 | 0 | 0 | 0 |
| 30 | | ++ | ++ | - | ? | 12045 | 25365 | 18546 | 0 | 4325 |
| 31 | 0,5+ | - | + | - | 4 | 1524 | 6528 | 0 | 0 | 1095 |
| 32 | | - | - | - | 6 | 39 | 128 | 0 | 0 | 0 |
| 33 | | +++ | +++ | 0,5+ | 142 | 9602 | 21795 | 11542 | 598 | 5695 |
| 34 | | +++ | +++ | +++ | 120 | 16325 | 35985 | 24065 | 11248 | 16524 |
| 35 | | +++ | +++ | +++ | 159 | 25639 | 45681 | 29032 | 15486 | 21468 |
| 36 | | +++ | +++ | ++ | 135 | 21025 | 32598 | 25584 | 12495 | 15421 |
| 37 | | +++ | + | - | ? | 15421 | 25625 | 20135 | 0 | 2154 |
| 38 | | +++ | +++ | +++ | 140 | 24569 | 38569 | 29584 | 18542 | 14025 |
| 39 | | +++ | +++ | 0,5+ | 132 | 12542 | 25146 | 14201 | 354 | 5698 |
| 40 | | +++ | +++ | +++ | ? | 19854 | 29524 | 21024 | 11245 | 9587 |
| | | | | | Valeur seuil: | 115 | 315 | 0 | 0 | 0 |

| | | | | | | | | | | |
|---|---|---|---|---|---|---|---|---|---|---|
| NB : dans ce Tableau, le point d'interrogation signifie que le test Abbott n'a pas été réalisé. | | | | | | | | | | |

Tous les sérums référencés positifs ont été retrouvés positifs en mettant en oeuvre les lames conformes à l'Invention.

De la même manière, tous les sérums référencés négatifs ont été retrouvés négatifs en mettant en oeuvre les lames conformes à l'Invention.

Les résultats montrent que le signal de fluorescence obtenu à faible puissance (P35, PMT50) est élevé et ne nécessite pas une seconde lecture à une plus forte puissance du scanner (L55, PMT 70).

Pour HCV Lot 1 et Lot 2, pour lesquels une faible valeur de fluorescence est observée pour les négatifs, la valeur seuil est calculée comme la moyenne de 20 sérums négatifs moins le bruit de fond, additionné de 3 fois l'écart type sur ces valeurs des sérums négatifs. Pour les autres antigènes testés, aucune fluorescence supérieure au bruit de fond n'est observée pour les sérums négatifs ; la valeur seuil correspond donc à la valeur des négatifs moins le bruit de fond, soit zéro

Ainsi, pour HCV core, NS4 et NS3, dès que l'on observe un signal de fluorescence, c'est que le sérum est positif. Ceci représente un énorme avantage par rapport à un sérodiagnostic effectué de manière classique par la méthode ELISA pour laquelle un signal non spécifique est toujours observé avec les sérums négatifs, ce qui nécessite de définir une valeur seuil qui est fonction de ce signal non spécifique.

De plus il ressort de ces résultats que le Lot 2 est particulièrement intéressant : il augmente la sensibilité de la séro-détection en permettant de détecter sans ambiguïté des sérums faibles en ELISA Abbott (inférieurs à 10), pour lesquels le RIBA (Recombinant Immunoblot Assay : test immunotransfert recombinant réalisé sur une membrane de nitrocellulose et qui permet de savoir contre quel antigène du virus de l'Hépatite C sont dirigés les anticorps produits suite à une infection virale) est soit positif soit limite (0,5+). L'utilisation de l'antigène du Lot 2 adsorbé sur un support semicarbazide permet donc d'améliorer la sensibilité de la détection par rapport à l'ELISA, ce qui est particulièrement intéressant en terme de précocité de la détection. De plus, les biopuces à polypeptides conformes à l'Invention sont plus spécifiques que l'ELISA Abbott de référence. En effet, des sérums faux positifs Abbott (sérums 2, 9, 17, 18, 25, 26, 29 et 32, sérums confirmés négatifs RIBA) ont été diagnostiqués correctement (négatifs) en utilisant les biopuces à polypeptides conformes à l'Invention.

Les lames de verres semicarbazides sur lesquelles sont adsorbés des antigènes NS3, NS4 et core constituent donc des outils de diagnostic sensibles et spécifiques permettant de différencier les différents antigènes de l'hépatite C alors qu'avec la technique ELISA, qui utilise des combinaisons de différents antigènes, il n'est pas possible d'obtenir une telle différenciation.

### EXEMPLE 5 : UTILISATION DE PUCES A POLYPEPTIDES SELON L'INVENTION POUR LE SÉRODIAGNOSTIC DU SIDA

### 1) Matériel et méthodes

Dans cet exemple, 30 dépôts par antigènes ont été réalisés sur chaque lame.

Deux antigènes différents du virus du SIDA (HIV) ont été testés, il s'agit des antigènes Gp41 et Gp120 qui sont des glycoprotéines d'enveloppe du virus. Ces antigènes HIV sont produits par Lily Bio-products, Hanan, Chine.

La protéine A a été utilisée comme témoin positif.

Les concentrations testées sont les suivantes :
- Gp41 : 2 ; 1 ; 0,5 : 0,1 et 0,05 mg/ml.
- Gp 120 : 0,5 ; 0,25 ; 0,1 et 0,05 mg/ml.

90 sérums référencés positifs et 20 sérums référencés négatifs ont été testés (technique ELISA : tests Ac HIV Genscreen Plus® de la société BIORAD et test HIV Integral de la société BEHRING).

### 2) Résultats

Les résultats obtenus sont reportés dans le Tableau III ci-après. Dans ce tableau, les valeurs données correspondent à la valeur de la fluorescence moins le bruit de fond moyen de la lame.

**TABLEAU III**

| Sérum | Biopuces | | | | | | | | | | ELISA |
|---|---|---|---|---|---|---|---|---|---|---|---|
| | Gp 120 | | | | | Gp 41 | | | | | |
| | 0,5 mg/ml | 0,25 mg/ml | 0,1 mg/ml | 0,05 mg/ml | 2 mg/ml | | mg/ml | 0,5 mg/ml | 0,1 mg/ml | 0,05 mg/ml | |
| 1 | 41730 | 45803 | 10773 | 7431 | 31803 | | 27549 | 22958 | 13036 | 8696 | positif |
| 2 | 41251 | 6674 | 8697 | 9715 | 8801 | | 17586 | 9713 | 4403 | 2753 | positif |
| 3 | 30864 | 25786 | 11666 | 10212 | 17026 | | 29694 | 24347 | 23607 | 18137 | positif |
| 4 | 32520 | 32206 | 11738 | 15973 | 11655 | | 17779 | 12531 | 6333 | 1276 | positif |
| 5 | 42022 | 28683 | 11415 | 12791 | 16405 | | 15092 | 8320 | 2425 | 416 | positif |
| 6 | 38148 | 28426 | 18753 | 12226 | 19196 | | 21076 | 16184 | 13239 | 10817 | positif |
| 7 | 40494 | 30883 | 10035 | 9086 | 6827 | | 23866 | 10504 | 9898 | 9131 | positif |
| 8 | 20017 | 10248 | 4870 | 1697 | 2542 | | 4125 | 1952 | 1476 | 624 | positif |
| 9 | 39166 | 33241 | 18446 | 19500 | 32768 | | 31779 | 16132 | 31833 | 22971 | positif |
| 10 | 30095 | 31628 | 28099 | 9779 | 26105 | | 9199 | 10716 | 24191 | 17998 | positif |
| 11 | 25325 | 32705 | 20765 | 6586 | 24592 | | 13347 | 13756 | 17951 | 17910 | positif |
| 12 | 31086 | 29523 | 20547 | 23421 | 31727 | | 9113 | 11450 | 16056 | 8712 | positif |
| 13 | 15770 | 9700 | 4111 | 2335 | 2754 | | 1025 | 1001 | 1069 | 558 | positif |
| 14 | 26899 | 20507 | 13152 | 5158 | 34566 | | 18887 | 17524 | 24524 | 19103 | positif |
| 15 | 40110 | 22294 | 14237 | 13483 | 23908 | | 10768 | 9704 | 12873 | 12875 | positif |
| 16 | 29285 | 14812 | 12227 | 13764 | 5741 | | 16013 | 10362 | 7814 | 4958 | positif |
| 17 | 6169 | 2828 | 2472 | 983 | 12809 | | 1337 | 543 | 536 | 372 | positif |
| 18 | 10737 | 5861 | 3221 | 847 | 19181 | | 3125 | 857 | 912 | 316 | positif |
| 19 | 3372 | 1710 | 1861 | 855 | 14325 | | 401 | 228 | 378 | 241 | positif |
| 20 | 4826 | 2600 | 1883 | 859 | 8209 | | 459 | 74 | 50 | 48 | positif |
| 21 | 9420 | 4764 | 2473 | 2481 | 18438 | | 530 | 420 | 558 | 447 | positif |
| 22 | 3716 | 1157 | 1488 | 687 | 11022 | | 3016 | 529 | 245 | 153 | positif |
| 23 | 3962 | 2142 | 1873 | 699 | 16695 | | 1965 | 1309 | 559 | 453 | positif |
| 24 | 4829 | 2160 | 2030 | 1818 | 23621 | | 8958 | 2658 | 1956 | 1401 | positif |
| 25 | 7940 | 4661 | 2528 | 1071 | 19965 | | 6998 | 6377 | 2316 | 1506 | positif |
| 26 | 21004 | 9097 | 5555 | 2889 | 19658 | | 3999 | 968 | 655 | 636 | positif |
| 27 | 2887 | 813 | 2055 | 355 | 11946 | | 1917 | 256 | 128 | 182 | positif |
| 28 | 25044 | 8379 | 10041 | 14256 | 33037 | | 29819 | 23874 | 5817 | 1251 | positif |
| 29 | 18541 | 7548 | 2514 | 544 | 17854 | | 14511 | 2512 | 956 | 251 | positif |
| 30 | 13921 | 8514 | 3357 | 1674 | 19126 | | 5050 | 1146 | 720 | 446 | positif |
| 31 | 4545 | 1698 | 1672 | 596 | 16584 | | 6584 | 3215 | 845 | 605 | positif |
| 32 | 15214 | 7996 | 4562 | 1996 | 21996 | | 8965 | 3915 | 1746 | 1748 | positif |
| 33 | 9862 | 4143 | 3326 | 1139 | 13948 | | 2438 | 1069 | 586 | 293 | positif |
| 34 | 2985 | 1042 | 1479 | 546 | 4299 | | 117 | 69 | 79 | 92 | positif |
| 35 | 1475 | 832 | 641 | 440 | 6033 | | 110 | 92 | 93 | 99 | positif |
| 36 | 1023 | 466 | 694 | 312 | 6999 | | 1012 | 332 | 123 | 134 | positif |
| 37 | 4667 | 3665 | 2296 | 1050 | 12348 | | 627 | 321 | 471 | 109 | positif |
| 38 | 19895 | 7985 | 6985 | 3965 | 31335 | | 12691 | 2965 | 2018 | 1824 | positif |
| 39 | 8262 | 3918 | 2217 | 2010 | 12766 | | 404 | 152 | 130 | 124 | positif |
| 40 | 8454 | 4512 | 2520 | 905 | 19958 | | 1985 | 1081 | 817 | 569 | positif |
| 41 | 4869 | 3154 | 2088 | 1876 | 14431 | | 301 | 174 | 108 | 162 | positif |
| 42 | 4418 | 1700 | 2009 | 706 | 17996 | | 2020 | 899 | 658 | 570 | positif |
| 43 | 14731 | 8722 | 3526 | 924 | 18234 | | 2762 | 1019 | 537 | 489 | positif |
| 44 | 5882 | 2186 | 1850 | 1108 | 10068 | | 3715 | 946 | 441 | 257 | positif |
| 45 | 5793 | 3095 | 2070 | 1401 | 23721 | | 7457 | 4045 | 1450 | 628 | positif |
| 46 | 1015 | 857 | 755 | 170 | 19449 | | 2928 | 2075 | 468 | 826 | positif |
| 47 | 12328 | 7366 | 3145 | 1399 | 22145 | | 2921 | 844 | 340 | 215 | positif |
| 48 | 5752 | 2618 | 2431 | 797 | 20449 | | 1014 | 661 | 673 | 431 | positif |
| 49 | 8488 | 3424 | 4210 | 1141 | 21470 | | 4823 | 4523 | 1853 | 713 | positif |
| 50 | 3989 | 1744 | 1346 | 619 | 18434 | | 6315 | 4185 | 1803 | 160 | positif |
| 51 | 903 | 650 | 671 | 291 | 658 | | 510 | 110 | 92 | 56 | positif |
| 52 | 6905 | 4325 | 2102 | 1998 | 11120 | | 3956 | 2481 | 442 | 517 | positif |
| 53 | 2939 | 2002 | 773 | 440 | 2669 | | 542 | 372 | 317 | 385 | positif |
| 54 | 6683 | 3295 | 1408 | 572 | 19008 | | 5583 | 3236 | 856 | 813 | positif |
| 55 | 1985 | 549 | 731 | 433 | 716 | | 250 | 225 | 234 | 212 | positif |
| 56 | 3228 | 1990 | 940 | 540 | 2070 | | 395 | 262 | 166 | 221 | positif |
| 57 | 12985 | 7665 | 2885 | 1655 | 10926 | | 3952 | 2365 | 699 | 548 | positif |
| 58 | 6254 | 3452 | 1958 | 1841 | 7985 | | 1682 | 676 | 487 | 707 | positif |
| 59 | 6428 | 3858 | 1754 | 1088 | 15985 | | 7542 | 4658 | 746 | 699 | positif |
| 60 | 9467 | 8235 | 2547 | 1024 | 17883 | | 10764 | 7289 | 1396 | 1127 | positif |
| 61 | 8260 | 6841 | 2403 | 1013 | 11923 | | 3895 | 3265 | 372 | 303 | positif |
| 62 | 6341 | 4937 | 2038 | 928 | 6143 | | 2699 | 2382 | 720 | 387 | positif |
| 63 | 4812 | 3236 | 1286 | 762 | 2828 | | 1311 | 950 | 154 | 188 | positif |
| 64 | 2446 | 1417 | 1388 | 659 | 1542 | | 631 | 541 | 138 | 194 | positif |
| 65 | 5896 | 3199 | 1838 | 709 | 1958 | | 424 | 197 | 74 | 80 | positif |
| 66 | 13101 | 8985 | 3652 | 1658 | 19856 | | 7910 | 5308 | 1921 | 1295 | positif |
| 67 | 5099 | 2003 | 1354 | 784 | 7985 | | 2654 | 1995 | 318 | 422 | positif |
| 68 | 8665 | 4980 | 1927 | 1396 | 17168 | | 8105 | 5520 | 1026 | 851 | positif |
| 69 | 12542 | 8956 | 499 | 542 | 9584 | | 5421 | 2865 | 254 | 310 | positif |
| 70 | 9895 | 4947 | 2695 | 990 | 13114 | | 4870 | 2991 | 1819 | 1399 | positif |
| 71 | 7149 | 5658 | 2476 | 2975 | 16003 | | 6859 | 3112 | 3722 | 1496 | positif |
| 72 | 6631 | 4666 | 2296 | 1312 | 8465 | | 681 | 572 | 310 | 418 | positif |
| 73 | 12220 | 11355 | 4037 | 2916 | 19856 | | 4685 | 3378 | 921 | 617 | positif |
| 74 | 9738 | 8170 | 2855 | 1298 | 12710 | | 2156 | 1985 | 1254 | 800 | positif |
| 75 | 9965 | 6847 | 2658 | 797 | 14587 | | 2654 | 1542 | 554 | 402 | positif |
| 76 | 5948 | 3772 | 1875 | 3695 | 13789 | | 1077 | 1069 | 329 | 611 | positif |
| 77 | 7958 | 3889 | 2006 | 744 | 7130 | | 386 | 348 | 162 | 202 | positif |
| 78 | 5811 | 4568 | 1935 | 1609 | 6700 | | 833 | 659 | 227 | 246 | positif |
| 79 | 3083 | 1738 | 1414 | 1551 | 5630 | | 245 | 242 | 169 | 201 | positif |
| 80 | 2546 | 1214 | 1021 | 356 | 1985 | | 1021 | 548 | 365 | 212 | positif |
| 81 | 10254 | 8457 | 5468 | 2154 | 15214 | | 2452 | 1025 | 566 | 213 | positif |
| 82 | 5621 | 2354 | 1214 | 548 | 14584 | | 1744 | 365 | 219 | 145 | positif |
| 83 | 5327 | 4098 | 1977 | 1129 | 6174 | | 240 | 390 | 269 | 250 | positif |
| 84 | 2635 | 1966 | 1600 | 691 | 1084 | | 167 | 116 | 107 | 77 | positif |
| 85 | 8769 | 6988 | 2371 | 1520 | 9906 | | 287 | 235 | 199 | 301 | positif |
| 86 | 1044 | 998 | 788 | 444 | 8955 | | 1699 | 842 | 432 | 509 | positif |
| 87 | 6126 | 4416 | 3234 | 2078 | 1326 | | 1181 | 86 | 104 | 76 | positif |
| 88 | 6356 | 3455 | 1893 | 1724 | 9098 | | 495 | 450 | 141 | 216 | positif |
| 89 | 17917 | 7762 | 4384 | 2328 | 16504 | | 3324 | 856 | 576 | 533 | positif |
| 90 | 5435 | 2495 | 2335 | 724 | 18104 | | 921 | 607 | 596 | 398 | positif |

NB : 20 sérums négatifs ont été testés ; ils ne donnent pas de valeur de fluorescence supérieure au bruit de fond (valeur seuil = 0).

Ces résultats montrent que la mise en oeuvre du test conforme à l'Invention permet un diagnostic sensible et spécifique à 100 % des sérums positifs et négatifs, à toutes les concentrations testées

En ce qui concerne l'antigène Gp41, la concentration optimale est de 1 mg/ml et de 0,5 mg/ml pour l'antigène Gp120, cependant pour ce dernier le plateau de saturation du signal n'a peut-être pas été atteint, ce qui laisse à supposer que des concentrations plus élevées pourraient également conduire à d'excellents résultats.

### EXEMPLE 6 : ÉTUDE DE LA STABILITÉ DANS LE TEMPS DES DISPOSITIFS PRÉPARÉS AUX EXEMPLES 4 ET 5

Afin de tester la stabilité dans le temps des dispositifs selon l'Invention, des lames de verre semicarbazides imprimées par adsorption d'antigènes du virus du SIDA, telles que préparées à l'exemple 5 ci-dessus, ont été placées à 37°C en atmosphère humide pendant une durée de 1 ou de 3 mois. Ces conditions permettent de réaliser une étude de vieillissement accéléré.

Cette étude a porté sur des lames préparées avec différents antigènes, à plusieurs concentrations :
- HIV Gp41 à 2 ; 1 ; 0,5 ; 0,1 et 0,05 mg/ml ;
- HIV Gp120 à 0,5 ; 0,25 ; 0,1 et 0,05 mg/ml ;

Les résultats obtenus sont reportés sur les figures 1 et 2 annexées sur lesquelles le signal de fluorescence est fonction de la concentration en antigènes, et ce à 0 (barres hachurées), 1 (barres à damiers) et 3 mois de vieillissement accéléré (barres unies).

Ces résultats mettent en évidence une excellente stabilité des lames après 3 mois de vieillissement accéléré, quelles que soient les concentrations testées.

### EXEMPLE 7 COMPARATIF : ÉTUDE DE LA STABILITÉ DES LAMES AMINÉES DE L'ART ANTÉRIEUR

Des lames de microscope silanisées avec du 3-aminopropyltriméthoxysilane telles que décrites par exemple dans l'article de Zammatteo N. *et al.,* Anal. Biochem., 2000, **280**, 143-150, ainsi que des lames fonctionnalisées par des groupements semicarbazides telles que décrites à l'exemple 1 ci-dessus, ont été imprimées avec deux antigènes du virus du SIDA (Gp120 et Gp41 tels que décrits ci-dessus à l'exemple 5), et ce à différentes concentrations (0,5 ; 0,25 ; 0,1 ; 0,05 et 0,01 mg/ml).

Les lames ainsi préparées ont été incubées avec 20 sérums référencés positifs. Les lames ainsi préparées ont été soumises à une étude de stabilité dans le temps, selon le protocole décrit ci-dessus à l'exemple 6.

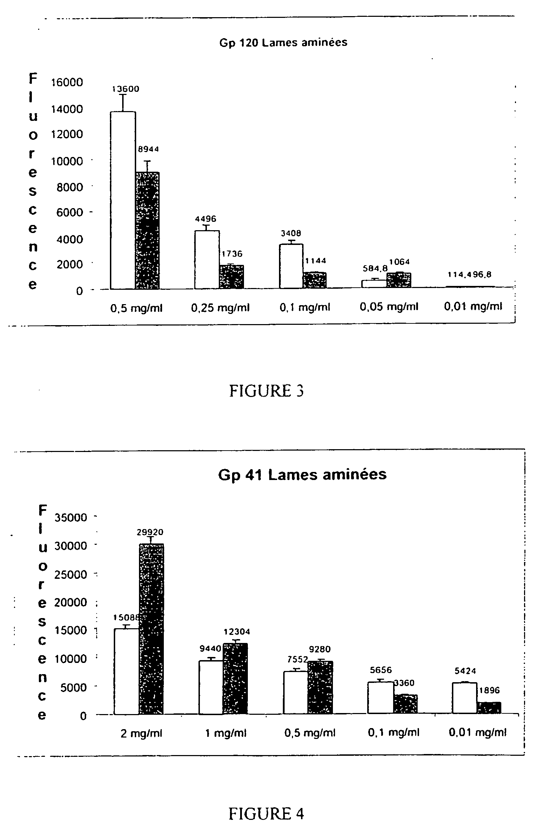

Les résultats obtenus sont représentés sur les figures 3 et 4 annexées sur lesquelles les valeurs de fluorescence sont exprimées en fonction des concentrations testées.

L'étude de stabilité a été arrêtée au bout de 30 jours (à t = 0 : barres claires et à t = 30 jours : barres grises).

Ces résultats montrent que les lames aminées de l'art antérieur ne sont pas stables après 1 mois de conservation ; on observe en effet des variations très importantes avec soit une chute de la fluorescence mesurée, soit son augmentation.

Par contre, et ainsi que cela a été démontré ci-dessus à l'exemple 6, les lames comportant des groupements semicarbazides conformes à l'Invention sont stables après trois mois de stockage en conditions accélérées.

### EXEMPLE 8 : UTILISATION DE LAMES SEMICARBAZIDES POUR UNE ÉTUDE DE MULTI SÉRO-DETECTION

Dans cet exemple, des lames de verres semicarbazides telles que préparées à l'exemple 1 ci-dessus, ont été imprimées avec des antigènes issus de différentes pathologies (HIV, hépatite B : HBV et hépatite C : HCV).

90 sérums référencés pour l'ensemble de ces pathologies par des tests classiques de type ELISA ont été testés sur les lames conformes à l'Invention (BIOPUCES).

Pour le test HIV, les méthodes de référence utilisées sont le test Ac HIV Genscreen Plus® de la société BIORAD et le test Ac HIV Integral de la société BEHRING. La positivité de ces tests est confirmée par un test Western Blot.

Pour les tests de l'hépatite B, les méthodes de référence utilisées sont le test HBs de la société BIORAD et le test HBc de la société BIORAD.

Pour les tests de l'hépatite C, les méthodes de référence utilisées sont le test HCV de la société BIORAD et le test HCV EIA 3.0 de la société ABBOTT. La positivité est confirmée par un test RIBA DECISCAN HCV PLUS®.

Pour le test de l'hépatite C sur les biopuces conformes à l'Invention, les lames ont été imprimées avec les antigènes recombinants de NS3 (0,5 mg/ml) et du gène entier (Lot 1 : 0,5 mg/ml).

Pour le test de l'hépatite B sur les biopuces conformes à l'Invention, les lames ont été imprimées avec les antigènes HBc (core) à 10 mg/ml et HBs Lot b à 5 mg/ml, tels que décrits ci-dessus à l'exemple 3.

Pour le test de la séropositivité par rapport au virus du SIDA sur les biopuces conformes à l'Invention, les lames ont été imprimées avec les antigènes Gp120 (0,5 mg/ml) et Gp41 (2 mg/ml) tels que décrits ci-dessus à l'exemple 5.

Avant leur utilisation, les sérums ont été dilués au 1/50^{ème} comme décrit précédemment. Les lames ont d'abord été incubées pendant 45 minutes avec ces sérums, puis pendant 45 minutes avec l'anticorps fluorescent complémentaire.

Les résultats obtenus sont reportés dans le Tableau IV ci-après :

**TABLEAU IV**

| | | ELISA | | | | BIOPUCE | | | | | |
|---|---|---|---|---|---|---|---|---|---|---|---|
| Entrée | **Nombre sérums** | **HIV** | **HBV** | | **HCV** | **HIV** | | **HBV** | | **HCV** | |
| | | | HBs | HBc | | Gp120 | Gp41 | HBs | HBc | Gene Entier | NS3 |
| **1** | 19 | + | - | - | - | + | + | - | - | - | - |
| **2** | 2 | + | - | + | - | + | + | - | + | - | - |
| **3** | 1 | + | - | - | + | + | + | - | - | + | + |
| **4** | 4 | - | - | - | + | - | - | - | - | - | - |
| **5** | 1 | + | - | - | + | + | + | - | - | - | - |
| **6** | 7 | - | - | + | + | - | - | - | + | + | + |
| **7** | 10 | - | - | - | + | - | - | - | - | + | + |
| **8** | 4 | - | + | + | - | - | - | + | + | - | - |
| **9** | 17 | - | - | + | - | - | - | - | + | - | - |
| **10** | 4 | - | - | + | + | - | - | - | + | + | + |
| **11** | 20 | - | - | - | - | - | - | - | - | - | - |

| | | | | | | | | | | | |
|---|---|---|---|---|---|---|---|---|---|---|---|
| NB: + sérum positif, - sérum négatif | | | | | | | | | | | |

Ces résultats montrent une corrélation parfaite pour 85 sérums testés sur 90 ; cependant pour les 5 sérums correspondant aux entrées 4 et 5, une différence a été observée entre les deux méthodes, ces sérums ont été trouvés HCV positifs par la méthode de référence ELISA et négatifs pour la méthode BIOPUCE conforme à l'Invention (entrées n°4 et n°5).

Afin de vérifier cette différence, le test RIBA -qui sert à confirmer la positivité d'un sérum vis-à-vis de l'hépatite C- a été réalisé et s'est révélé être négatif. Ces sérums sont donc HCV négatifs puisqu'ils ne possèdent pas d'anticorps dirigés contre le virus, ils n'ont donc pas été en contact avec ce virus.

Ceci confirme le résultat BIOPUCE qui avait donné ces sérums comme étant négatifs, ces sérums sont donc des faux-positifs pour la méthode de référence ELISA.

### EXEMPLE 9 : UTILISATION DE LAMES SEMICARBAZIDES POUR LE DOSAGE DE LA PROTÉINE HBs DANS UN MILIEU BIOLOGIOUE COMPLEXE

Cet exemple a pour but de démontrer que la mise en oeuvre des dispositifs conformes à l'Invention permet de fixer un anticorps monoclonal sur une lame de verre comportant des groupements semicarbazides (antigène dirigé contre l'antigène de surface du virus de l'hépatite B) qui est ensuite mise en contact avec un sérum possédant cet antigène de surface ; la révélation se faisant en utilisant un autre anticorps monoclonal marqué par de la rhodamine et dirigé contre l'antigène de surface du virus de l'hépatite B.

Cette technique dite de "sandwich" permet de détecter des antigènes circulants dans un sérum. Cette technique a de nombreuses applications potentielles, aussi bien en sérodiagnostic qu'en criblage de molécules biologiques (recherche de substances actives, de toxiques), en fixant toute une batterie d'anticorps monoclonaux connus et dirigés contre ces molécules biologiques.

### 1) Matériel et méthode

Des lames de verre préalablement fonctionnalisées par des groupements semicarbazide selon l'exemple 1 ci-dessus sont imprimées, à l'aide d'un spotteur automatique avec une solution contenant 3 mg/ml d'un anticorps monoclonal (anticorps murin clone NE3 de la société Advanced Immuno Chemical) dans un tampon carbonate/bicarbonate (pH 9,2).

Chaque dépôt est réalisé 3 fois, ce qui fait un total de 27 dépôts de solution d'anticorps monoclonal sur chaque lame.

On ajoute, à 200 µl de sérums négatifs dilués au 1/50^{ème}, des quantités connues d'un antigène HBs recombinant (Société Advanced Immuno Chemical à 0,5 mg/ml). Ces sérums (100 µl) sont ensuite mis en contact avec les lames de verre sur lesquelles les anticorps monoclonaux ont préalablement été fixés. Les dépôts sont recouverts par une lamelle couvre-objet. Les lames de verre sont ensuite incubées pendant 2 heures à 37°C en atmosphère humide.

Après lavages, l'étape de révélation est réalisée avec 100 µl d'une solution d'anticorps monoclonal murin anti-antigène de surface de l'hépatite B (clone NF5 commercialisé par la société Advanced Immuno Chemical à une concentration de 1,5 mg/ml), préalablement marqué à la rhodamine et dilué au 1/100^{ème} dans une solution de dilution (PBS-Tween 0,1 % - lait demi-écrémé 5%).

L'incubation est effectuée pendant 60 minutes entre lame et lamelle, à 37°C sous atmosphère humide.

Après lavages, les lames sont lues par le Scanner de la société Affymetrix, (Affymetrix 418 Array) à la puissance de P70, PMT90. La quantification de la fluorescence est effectuée à l'aide du logiciel Scanalyse® (Standford-University Software).

### 2) Résultats

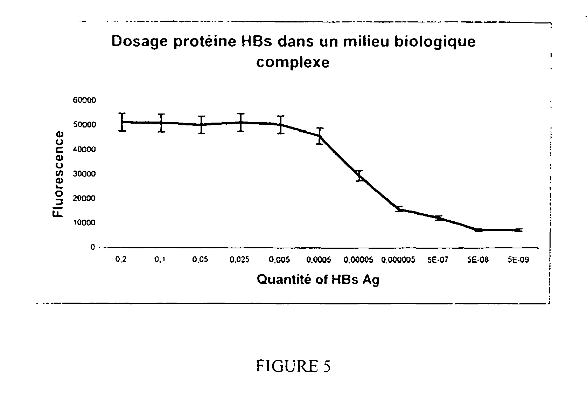

Les résultats obtenus figurent sur la figure 5 annexée sur laquelle la fluorescence est exprimée en fonction de la quantité d'antigène HBs recombinant ajoutée en mg/µl.

Ces résultats montrent que l'utilisation des dispositifs conformes à l'Invention permet de détecter des antigènes circulants. Ils montrent également que l'adsorption de l'anticorps sur la lame de verre permet de préserver l'accessibilité du site de fixation de l'antigène (segment Fab de l'anticorps) pour la reconnaissance antigène-anticorps.

## Revendications

1. Dispositif de présentation de polypeptides **caractérisé par le fait qu'**il comprend au moins un support solide fonctionnalisé par des groupements semicarbazides sur lesquels sont adsorbés lesdits polypeptides.

2. Dispositif selon la revendication 1, **caractérisé par le fait que** les polypeptides sont choisis parmi les peptides comprenant au moins deux acides aminés, les peptidomimétiques, les protéines et les fragments de protéines.

3. Dispositif selon la revendication 1 ou 2, **caractérisé par le fait que** le support solide est un matériau organique ou inorganique choisi parmi le verre, le silicium et ses dérivés et les polymères synthétiques.

4. Procédé de préparation d'un dispositif de présentation de polypeptides tel que défini à l'une quelconque des revendications 1 à 4, **caractérisé par le fait qu'**il comprend les étapes suivantes :
- la fonctionnalisation d'un support solide par des groupements semicarbazides, et
- le dépôt sous forme de spots d'échantillons de polypeptides et leur adsorption sur le support ainsi fonctionnalisé.

5. Procédé selon la revendication 4, **caractérisé par le fait que** la fonctionnalisation du support comprend :
- l'introduction d'une fonction amine par une réaction de silanisation du support,
- la transformation de la fonction amine en fonction isocyanate,
- la réaction de la fonction isocyanate avec un dérivé d'hydrazine pour former le groupement semicarbazide.

6. Procédé selon la revendication 4, **caractérisé par le fait que** la fonctionnalisation du support est effectuée en une seule étape, par réaction du support avec un silane portant un groupement semicarbazide.

7. Procédé selon l'une quelconque des revendications 4 à 6, **caractérisé par le fait que** le dépôt des polypeptides comprend :
- la préparation de solutions de polypeptides dans un tampon, à une concentration comprise entre 10 mg/ml et 0,01 mg/ml ;
- leur distribution dans un récipient approprié à leur prélèvement, de type puits de plaque de microtitration ;
- leur prélèvement à l'aide d'un appareil de prélèvement manuel ou automatisé, par exemple de type "spotteur" ;
- leur dépôt sur le support semicarbazide ; et éventuellement
- la saturation du support.

8. Utilisation d'un dispositif de présentation de polypeptides selon l'une quelconque des revendications 1 à 3, comme puces à polypeptides en tant qu'outil de diagnostic miniaturisé et hautement parallèle.

9. Utilisation d'un dispositif de présentation de polypeptides selon l'une quelconque des revendications 1 à 3, comme puces à polypeptides pour la détection d'un risque lors d'une transfusion ou d'un don d'organe.

10. Utilisation d'un dispositif de présentation de polypeptides selon l'une quelconque des revendications 1 à 3, comme puces à polypeptides pour le sérotypage ou le criblage d'épitopes.

11. Utilisation selon l'une quelconque des revendications 8 à 10, **caractérisée par le fait qu'**elle met en jeu la détection de réponses de type antigène-anticorps par l'utilisation de réactifs marqués, fluorescents, radioactifs ou marqués chimiquement.

12. Utilisation d'un dispositif de présentation de polypeptides selon l'une quelconque des revendications 1 à 3, comme puces à polypeptides pour la quantification de protéines dans des milieux biologiques complexes.

13. Utilisation d'un dispositif de présentation de polypeptides selon l'une quelconque des revendications 1 à 3, comme puces à polypeptides pour l'analyse des relations entre molécules biologiques peptidiques, de type ligand-récepteur.

## Claims

1. Device for presenting polypeptides, **characterised in that** it comprises at least one solid support functionalised by semicarbazide groups onto which said polypeptides are adsorbed.

2. Device according to claim 1, **characterised in that** the polypeptides are selected from among peptides comprising at least two amino acids, peptidomimetics, proteins and protein fragments.

3. Device according to claim 1 or 2, **characterised in that** the solid support is an organic or inorganic material selected from among glass, silicon and the derivatives thereof and synthetic polymers.

4. Process for preparing a device for presenting polypeptides as defined in any one of claims 1 to 4, **characterised in that** it comprises the following steps:
- functionalising a solid support with semicarbazide groups, and
- depositing polypeptide samples in the form of spots and adsorbing them on the support thus functionalised.

5. Process according to claim 4, **characterised in that** functionalising the support comprises:
- introducing an amine function by a reaction of silanisation of the support,
- converting the amine function into an isocyanate function,
- reacting the isocyanate function with a hydrazine derivative to form the semicarbazide group.

6. Process according to claim 4, **characterised in that** the functionalising of the support is carried out in a single step, by reacting the support with a silane carrying a semicarbazide group.

7. Process according to any one of claims 4 to 6, **characterised in that** the depositing of the polypeptides comprises:
- preparing solutions of polypeptides in a buffer at a concentration of between 10 mg/ml and 0.01 mg/ml;
- distributing them in a container suitable for sampling them, such as a microtitre plate well;
- sampling them using a manual or automatic sampling device, e.g. of the "spotter" type;
- depositing them on the semicarbazide support; and optionally
- saturating the support.

8. Use of a device for presenting polypeptides according to any one of claims 1 to 3 as polypeptide chips for use as a miniaturised and highly parallel diagnostic tool.

9. Use of a device for presenting polypeptides according to any one of claims 1 to 3, as polypeptide chips for detecting risks during transfusion or organ donation.

10. Use of a device for presenting polypeptides according to any one of claims 1 to 3, as polypeptide chips for serotyping or epitope screening.

11. Use of any one of claims 8 to 10, **characterised in that** it comprises detecting antigen-antibody type responses by the use of labelled, fluorescent, radioactive or chemically labelled reagents.

12. Use of a device for presenting polypeptides according to any one of claims 1 to 3, as polypeptide chips for quantifying proteins in complex biological media.

13. Use of a device for presenting polypeptides according to any one of claims 1 to 3, as polypeptide chips for analysing relationships between peptidic biological molecules, of the ligand-receptor type.

## Patentansprüche

1. Vorrichtung zur Präsentation von Polypeptiden, **dadurch gekennzeichnet, dass** sie wenigstens einen festen Träger, der durch Semicarbazidgruppierungen funktionalisiert ist, umfasst, an den die Polypeptide adsorbiert sind.

2. Vorrichtung nach Anspruch 1, **dadurch gekennzeichnet, dass** die Polypeptide unter den Peptiden, die wenigstens zwei Aminosäuren umfassen, den Peptidomimetika, den Proteinen und den Proteinfragmenten ausgewählt sind.

3. Vorrichtung nach Anspruch 1 oder 2, **dadurch gekennzeichnet, dass** der feste Träger ein organisches oder anorganisches Material ist, das aus Glas, Silicium und seinen Derivaten und synthetischen Polymeren ausgewählt ist.

4. Verfahren zur Herstellung einer Vorrichtung zur Präsentation von Polypeptiden, wie sie in einem der Ansprüche 1 bis 3 definiert ist, **dadurch gekennzeichnet, dass** es die folgenden Stufen umfasst:
- Funktionalisierung eines festen Trägers durch Semicarbazidgruppierungen und
- Abscheidung von Polypeptiden in Form von Probenpunkten und ihre Adsorption auf dem so funktionalisierten Träger.

5. Verfahren nach Anspruch 4, **dadurch gekennzeichnet, dass** die Funktionalisierung des Trägers umfasst:
- Einführung einer Aminfunktion durch eine Silanisierungsreaktion des Trägers,
- Transformation der Aminfunktion in eine Isocyanatfunktion,
- Reaktion der Isocyanatfunktion mit einem Hydrazinderivat zur Bildung der Semicarbazidgruppierung.

6. Verfahren nach Anspruch 4, **dadurch gekennzeichnet, dass** die Funktionalisierung des Trägers in einer einzigen Stufe durch Reaktion des Trägers mit einem Silan, das eine Semicarbazidgruppierung trägt, durchgeführt wird.

7. Verfahren nach einem der Ansprüche 4 bis 6, **dadurch gekennzeichnet, dass** die Abscheidung der Polypeptide umfasst:
- Herstellung von Polypeptidlösungen in einem Puffer mit einer Konzentration zwischen 10 mg/ml und 0,01 mg/ml;
- ihre Verteilung in einem zu ihrer Entnahme geeigneten Behälter vom Typ Vertiefungen einer Mikrotiterplatte;
- ihre Entnahme mit Hilfe einer manuellen Entnahmeapparatur oder einer automatischen Entnahmeapparatur, bspw. vom Spotter-Typ;
- ihre Abscheidung auf dem Semicarbazidträger und ggf.
- Sättigung des Trägers.

8. Verwendung einer Vorrichtung zur Präsentation von Polypeptiden nach einem der Ansprüche 1 bis 3 als Polypeptid-Chips als Diagnosewerkzeug, das miniaturisiert und hoch parallel ist.

9. Verwendung einer Vorrichtung zur Präsentation von Polypeptiden nach einem der Ansprüche 1 bis 3 als Polypeptid-Chips für die Detektion eines Risikos bei einer Transfusion oder einer Organspende.

10. Verwendung einer Vorrichtung zur Präsentation von Polypeptiden nach einem der Ansprüche 1 bis 3 als Polypeptid-Chips für die Serumtypisierung oder das Screening von Epitopen.

11. Verwendung nach einem der Ansprüche 8 bis 10, **dadurch gekennzeichnet, dass** sie Detektion von Antworten des Antigen-Antikörpertyps durch Verwendung von markierten Reagenzien, fluoreszierenden Reagenzien, radioaktiven Reagenzien oder chemisch markierten Reagenzien einsetzt.

12. Verwendung einer Vorrichtung zur Präsentation von Polypeptiden nach einem der Ansprüche 1 bis 3 als Polypeptid-Chips für die quantitative Bestimmung von Proteinen in komplexen biologischen Medien.

13. Verwendung einer Vorrichtung zur Präsentation von Polypeptiden nach einem der Ansprüche 1 bis 3 als Polypeptid-Chips zur Analyse von Ligand-Rezeptor-Beziehungen zwischen biologischen Peptidmolekülen.
